# EUROPEAN PATENT SPECIFICATION

(11) **EP 2 723 234 B1**
(45) Date of publication and mention of the grant of the patent: **08.04.2015**
(21) Application number: 12741405.0
(22) Date of filing: 20.06.2012
(51) Int. Cl.: A61B 5/145, A61B 5/1495, A61B 5/15, A61B 5/151, A61B 5/157

(54) **Analyte testing device with lancet cartridge and test strip cartridge**
Analyttestvorrichtung mit Lanzettenmagazin und Teststreifenmagazin
Dispositif d'essai d'analytes avec cartouche de lancette et cartouche de bandelette d'essai

(30) Priority: 21.06.2011 US 201113165621; 30.11.2011 US 201113307364
(43) Date of publication of application: 30.04.2014
(73) Proprietor: Yofimeter, LLC, La Jolla, California 92037 (US)
(72) Inventor: SHAANAN, Gad, La Jolla, California 92037 (US); GOLDMAN, Marc, La Jolla, California 92037 (US)
(74) Representative: Mellgren, Maria Charlotta
(86) International application number: PCT/US2012/043331
(87) International publication number: WO 2012/177748

(56) References cited:
- WO-A1-2010/040089
- WO-A2-2009/027950
- US-A1- 2010 331 653
- US-B2- 7 922 971

## Description

### Field of the Invention

The field of the invention is analyte testing devices.

### Background

Analyte testing devices play a critical roll in modem diagnosis and management of health-related issues. For example, a sample of human blood, urine, and/or saliva can be tested for glucose levels, fructosamine, hematocrit, hemoglobin blood oxygen saturation, lactates, iron, pH, cholesterol, liver enzymes (AST, ALT, alkaline phosphatase / GGT, LDH, bilirubin, etc), hormones, and other chemicals.

For many diabetic patients, monitoring glucose levels and administering appropriate insulin dosages is a daily activity that requires a significant amount of time and mental energy. Current glucose meters and lancing devices often involve multiple devices, components, and supplies, and require numerous steps to monitor glucose levels. For example, conventional glucose monitoring systems may require numerous steps involving reading a test strip, readying a lancet, using the lancet, putting blood on the test strip and inserting the strip into the glucose meter, reading data from a meter, recording the data in a journal and remembering to bring the journal to the next doctor visit, and then putting away the strip and lancet packages, disposing of loose components, and storing the glucose meter. Thus, there is a need to reduce steps and simplify devices and supplies for monitoring analytes. Other needs include a compact analyte testing device and hands-free disposal of test strips.

Several known prior art references are directed at simplifying the devices and processes for monitoring analytes. Significantly, however, the prior art systems each appear to address only a subset of the convenience issues. US Patent No. 6472220 to Simons, for example, discloses an integrated lancing device and glucose meter. The device holds a cassette that stores a plurality of lancets and test strips. Each lancet is paired with a test strip into a single integrated unit, thus simplifying the number of separate supply components. Unfortunately, the test strip-lancet unit contemplated in Simons prevents the user from using lancets independently of the test strips.

US Patent No. 7192405 to DeNuzzio also provides an integrated lancet-test strip unit, similar to Simons. DeNuzzio suffers from the same drawbacks as Simons.

US Patent No. 7582063 to Wurster discloses a glucose meter that houses a carrier of test strips and a carrier of lancets. The carriers must be rotated into position each time a new blood test is performed. Wurster fails to provide a device that advances the next lancet into position, cocks a lancing device, and partially exposes a test strip for use, all with a single motion. Furthermore, the two carriers are joined together, thus preventing the lancets from being used independently of the test strips.

US Patent No. 4794926 to Munsch discloses a lancing device that holds a cartridge with a plurality of lancets. Rotating the cartridge in the lancing device simultaneously loads the next lancet into position for ejection while "cocking" the lancet for ejection. However, Munsch fails to integrate the lancing device with a glucose meter, and also fails to partially expose a test strip when the lancet cartridge is rotated. WO 2010/040089 discloses an integrated lancing and analyte measuring system which minimizes the number of actions required to operate the system, accomplished in part by combining two or more steps in one.

WO 2009/027950 discloses an apparatus comprising a common housing for a sampling means, a determining means and an injecting means, a switching means for switching between any of the sampling mode, determining mode and the injecting mode, and actuation means for individually actuating at least one of the sampling means, determining means and the injecting means.

US 2010/331653 discloses in vitro analyte meters that include a meter portion that is moveable relative to at least one other meter portion. Embodiments include moveable meters that are integrated with in vivo analyte systems.

US Patent Nos. 7922971, 6997343, 7211096, and 6616616 are other examples of known references that attempt to simplify methods and devices for monitoring glucose levels.

The POGO^{™} System by Intuity Medical, Inc. (see http://www.presspogo.com/pogo/system/) is a commercially available glucose and lancing device that is designed to simplify glucose monitoring. While the POGO^{™} System reduces the steps and components required and is an improvement over conventional systems, the POGO system fails to provide a separate lancet cartridge and test strip cartridge. As such, the user cannot use lancets independently of test strips.

It has yet to be appreciated that an analyte testing device can house a lancet cartridge and a separate test strip cartridge. Furthermore, it has yet to be appreciated that an analyte testing device can be configured to advance a lancet cartridge, cock a lancing device, and expose a test strip, all in a single motion.

Thus, there is still a need for an integrated analyte testing device that reduces the components and steps required for monitoring analyte levels.

The inventive subject matter provides apparatus, systems and methods in which an analyte testing device has a housing that removeably holds a lancet cartridge and a test strip cartridge. Each of the lancet and test strip cartridges holds a plurality of lancets and test strips, respectively. The testing device also has an conversion electronics, which receives a signal from the test trip and converts the signal into readable data.

In one aspect of preferred embodiments, the testing device has an actuator configured to (i) cock a lancing device, (ii) expose individual ones of the plurality of test strips for use; and (iii) advance the lancet cartridge. Preferably, the actuator accomplishes all of these steps in a single action, i.e., with a single motion by the user.

In another aspect of preferred embodiments, the testing device has a disengaging mechanism configured to disengage the second cartridge from the actuator. In this manner, the individual test strips can be used independently of the lancets. Optionally, a second disengaging mechanism can be included to disengage the first cartridge from the actuator.

In yet other aspects of preferred embodiments, the testing device further includes a wireless transmitter for transmitting data to an external device. The testing device can also include a processor programmed to time-stamp data, keep track of supplies ordered and used, supplies remaining in a personal inventory (e.g., home closet), automatically re-order supplies, evaluate data, and send notifications as a function of the data.

From a method perspective, the testing device can be used by: (1) inserting a lancet cartridge into the device; (2) inserting a test strip cartridge into the device; (3) cocking a lever on the device, which operates a mechanism that (a) cocks a lancing device, (b) exposes a test strip, and (c) advances the lancet cartridge; (4) deploying the lancing device in order to prick a body part and draw a blood sample; and (5) contacting the test strip to the blood sample.

Preferred methods further include reading an output of the device; ejecting and disposing of the test strip; replacing the lancet cartridge with a refill lancet cartridge; and replacing the test strip cartridge with a refill test strip cartridge.

Various objects, features, aspects and advantages of the inventive subject matter will become more apparent from the following detailed description of preferred embodiments, along with the accompanying drawing figures in which like numerals represent like components.

### Brief Description of The Drawing

Fig. 1 is a top perspective view of one embodiment of an analyte testing device.
Fig. 2 is the device of Fig. 1, wherein a test strip has been partially exposed.
Fig. 3 is a bottom perspective view of the device of Fig. 1.
Figs. 4a and 4b are perspective and side views, respectively, of one embodiment of a lancet cartridge.
Figs. 5a and 5b are perspective and side views, respectively, of one embodiment of a test strip cartridge.
Fig. 6 is a perspective view of one embodiment of a test strip.
Fig. 7 is a side view of the device of Fig. 1, showing the inner components of the device.
Fig. 8 is a side view of the device of Fig. 1, showing the inner components of the device as an actuator is being cocked.
Fig. 9 is a side view of the device of Fig. 1, showing the inner compartments of the device after an actuator has been cocked.
Fig 10 shows one embodiment of a linkage mechanism for the device of Fig. 1 at four different stages of use.
Fig. 11 is one embodiment of a linkage mechanism for the device of Fig. 1, coupled with the lancet cartridge of Fig. 4 and the test strip cartridge of Fig. 5.
Fig. 12 is a perspective view of one embodiment of an analyte testing device having an LCD touch-screen display.
Fig. 13 is perspective view of the device of Fig. 1 transmitting wireless communications to a laptop.
Fig. 14 is a perspective view of one embodiment of a docking station coupled with the device of Fig. 1.
Figs. 15a and 15b show one embodiment of an analyte testing device with a no-contact test strip ejecting mechanism.
Figs. 16a and 16b show perspective views of one embodiment of an analyte testing device with a wheel for adjusting lancet puncture depth and window for displaying depth setting.
Fig. 17 is a side view of an alternative embodiment of a lancet cartridge.
Fig. 18 is a schematic of one embodiment of a method of using the device of Fig. 1.

### Detailed Description

The following discussion provides many example embodiments of the inventive subject matter.

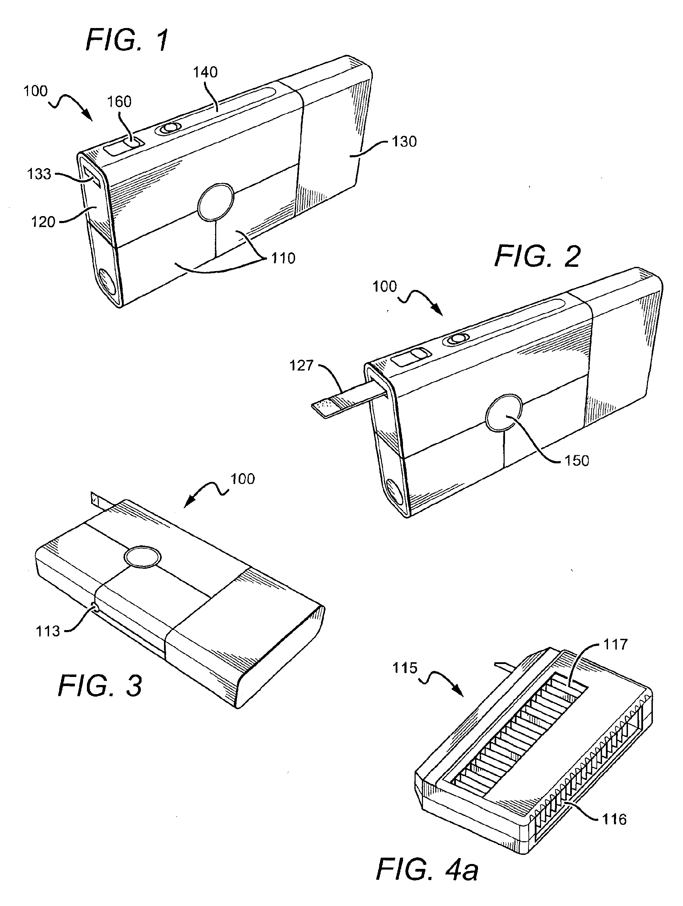

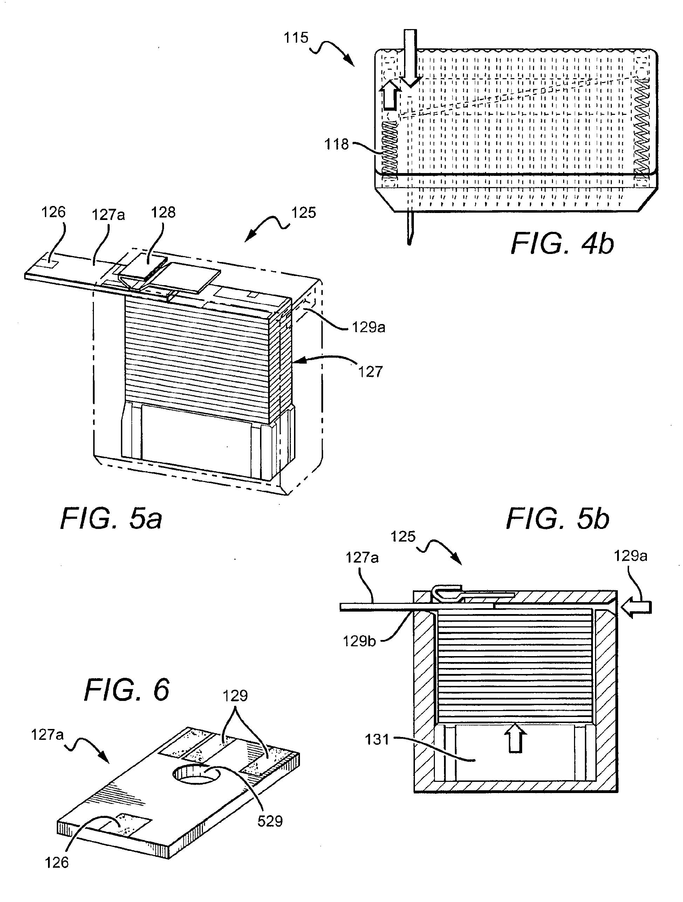

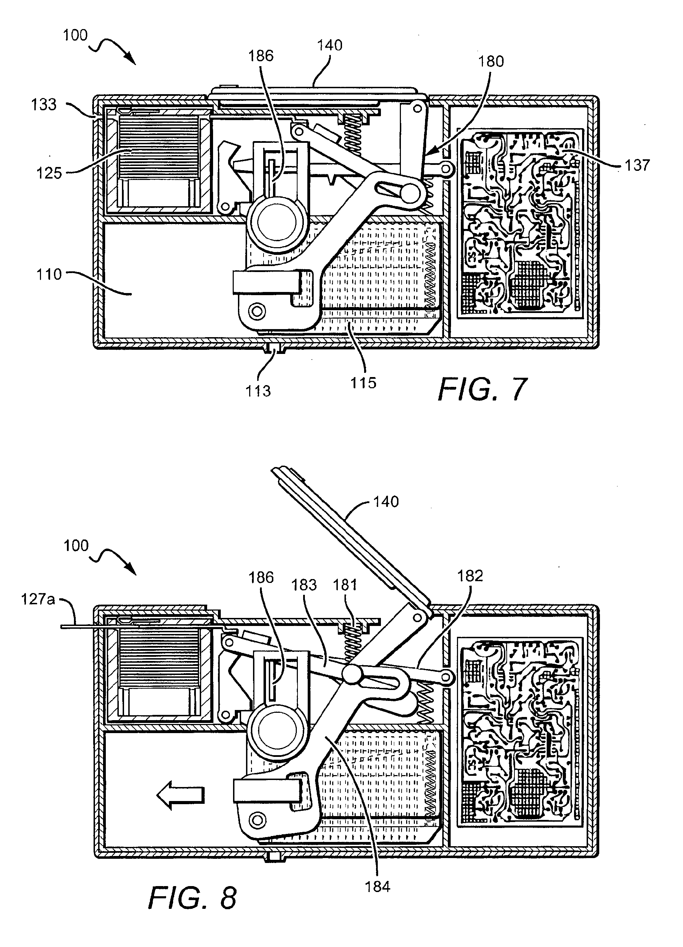

**Figure 1** shows an analyte testing device 100. The housing of device 100 has a first compartment 110 and a second compartment 120, for storing a lancet cartridge 115 (see Figures 4a and 4b) and a test strip cartridge 125 (see Figures 5a and 5b), respectively. Device 100 also has an electronics compartment 130 for housing conversion electronics 137 (see Figure 7). Conversion electronics 137 are communicatively coupled to an analyte sensor 126 (see Figure 5a and 6) within cartridge 125. Conversion electronics 137 is configured to convert a signal from analyte sensor 126 into readable data (e.g., glucose levels).

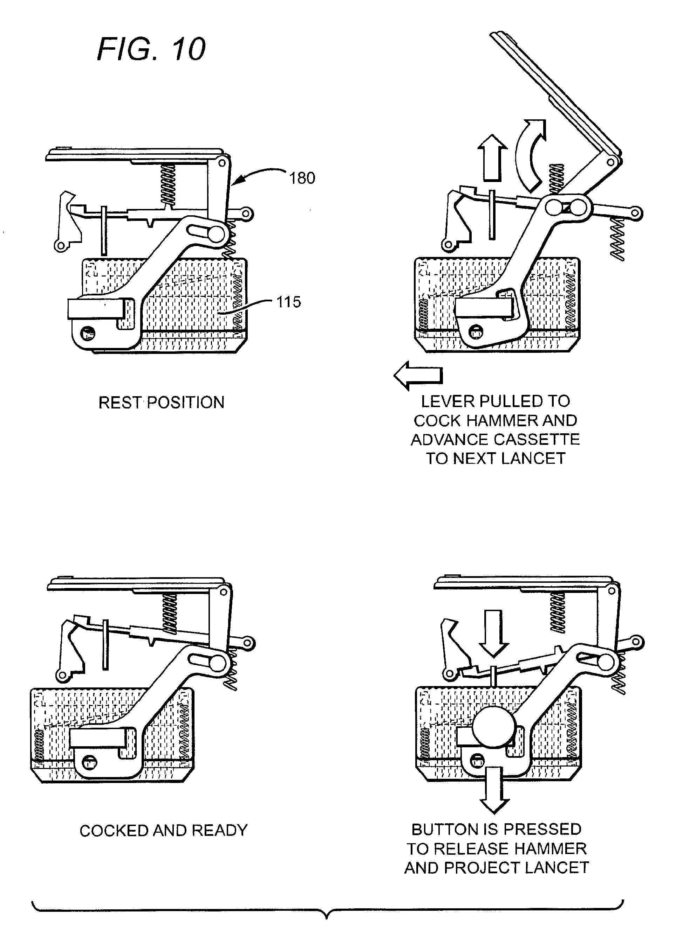

Device 100 has an actuator 140 configured to (i) cock a lancing device (see Figures 10-11) within device 100, (ii) expose a test strip for use, and (iii) advance the lancet cartridge. Device 100 also has a test strip ejector 160, which advantageously allows for hands-free disposal of a used test strip.

The housing of device 100 can be made of plastic, metal, composite, or any other material with structural and mechanical properties suitable for housing a lancet cartridge, test strip cartridge, electronics, and a linkage mechanism. Device 100 is preferably compact, with a height no more than 50 mm, a width no more than 17 mm, and a length no more than 100 mm. In especially preferred embodiments, the height is no more than 40 mm, a width is no more than 12 mm, and a length is no more than 75 mm. In some preferred embodiments, the housing of device 100 comprises an outer protective shell made of molded plastic and an inner desiccant liner to minimize exposure to moisture.

All ranges set forth herein should be interpreted as being inclusive of their endpoints.

Conversion electronics 137 preferably includes a facility for transmitting data and information out of the housing and to an external storage device (*e.g*., docking station, laptop, smart phone). It is contemplated that the data can be transmitted using a wireless protocol, and can even transmit data using two or more wireless protocols. Wired protocols and methods are also contemplated.

It is also contemplated that conversion electronics 137 can include a processor programmed to correlate individual instances of data and information derived from the data with time stamps. The processor can also be programmed to correlate individual instances of data with user-entered information such as voice recordings or text. In addition, the processor can be programmed to make an evaluation of the data and information, and send a notification to different recipients as a function of the evaluation. Yet still, the processor can be programmed to keep track of inventory of lancets and test strips, both within and outside the device, and re-order supplies as needed.

In other aspects of preferred embodiments, conversion electronics 137 includes a processor programmed to store voice recordings of diary information selected from the group consisting of supplies used or ordered, food eaten, exercise, medication taken, and estimated calories burned. The processor is also preferably programmed to prompt a user to use the device according to a pre-selected time and/or pre-selected time interval. Alternatively, electronics 137 can be equipped with an accelerometer or pedometer for measuring and calculating distance traveled and calories burned.

Conversion electronics 137 can also include a pedometer and/or accelerometer for calculating a distance traveled and calories burned. In such embodiments, electronics 137 preferably includes a processor for time-stamping the distances traveled and calories burned, and correlating this data with analyte test data.

**Figure 2** shows device 100 after actuator 140 has been cocked. The cocking of actuator 140 has caused a test strip 127 to be partially exposed or ejected from a slot 133 of device 100. Once actuator 140 has been cocked, a lancing device within device 100 can be deployed by pressing button 150, causing one of a plurality of lancets 117 (see Figures 4a and 4b) to exit from hole 113 as shown in **Figure 3**. The operation of device 100 will become more apparent from a discussion of figures 8-11.

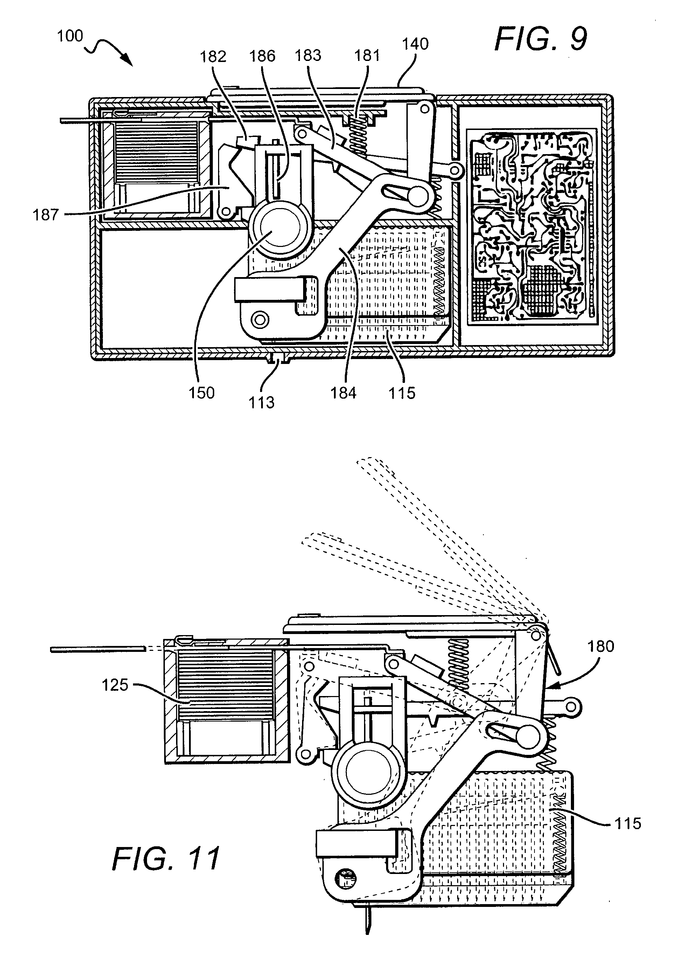

**Figures 4a** and **4b** show different views of a lancet cartridge 115. Cartridge 115 holds a plurality of lancets 117. A slot 116 is disposed on a side of cartridge 115, so that a hammer 186 (see Figures 9 and 10) of a lancing device within device 100 can contact one of the plurality of lancets 117, thus causing the lancet to partially exit cartridge 115. Optional spring 118 operates to retract the lancet back into cartridge 115. In other versions, the spring or equivalent retracting mechanism could be external to the cartridges (not shown).

Cartridge 115 can include any appropriate number of lancets, preferably between 15 and 25 lancets, more preferably between 18 and 22 lancets, and most preferably 20 lancets. The lancets are preferably sterilized and sealed prior to use.

**Figures 5a** and **5b** show different views of a test strip cartridge 125. Cartridge 125 holds a plurality of test strips 127. Cartridge 125 has electrical contacts 128 for communicatively coupling the test strips 127 to the conversion electronics 137. Alternatively, contacts 128 could be eliminated and electronics 137 could directly interface with contacts 129 of test strip 127 (see Figure 6) via an open aperture in cartridge 125. Cartridge 125 also has a slot 129a that couples with a test strip advance mechanism of device 100. When actuator 140 is cocked, the advance mechanism enters slot 129a and pushes a portion of test strip 127a out of slot 129b.

Cartridge 125 can include any appropriate number of test strips, preferably between 15 and 25 test strips, more preferably between 18 and 22 test strips, and most preferably 20 test strips.

Cartridge 125 preferably includes test strips configured to test for different analytes. For example, some test strips may test for glucose while other test strips test for fructosamine. Furthermore, cartridge 125 can have at least one test strip capable of testing for two analytes simultaneously, either by including two analyte-binding chemicals/reactants within one absorbing material or by including two different analyte sensors on one test strip. In addition, cartridge 125 preferably includes at least one calibration test strip for verifying the calibration of conversion electronics 137. In one embodiment, the calibration test strip is an analyte sensor that has a known amount of glucose.

Cartridge 125 also has a spring-load base 131 configured to push the plurality of test strips 127 upward, thus repositioning a new test strip into place after test strip 127a is removed from cartridge 125.

**Figure 6** shows a test strip 127a having an analyte sensor 126. Analyte sensors are well known and generally comprise an absorbent material with an analyte-binding reactant. Analyte sensor 126 is configured to generate a signal that is sent to electrical contacts 129. Electrical contacts 129 are communicatively coupled with electrical contacts 128 of cartridge 125 and allows the signal generated by analyte sensor 126 to reach the conversion electronics 137 for analysis. Alternatively, electrical contacts 129 could directly interface with conversion electronics 137 via an open aperture on cartridge 125.

**Figure 7** is a side view of device 100 showing various internal components of device 100. Figures 7 also shows the location of linkage 180 before actuator 140 has been cocked. **Figure 8** shows the position of linkage 180 as actuator 140 is being cocked.

**Figure 9** shows the position of linkage 180 after actuator 140 has been cocked. Linkage 180 is configured to function as a lancing device, a test strip advance mechanism, and a lancet cartridge advance mechanism. As actuator 140 is cocked upward, a link 182 is brought into juxtaposing contact with a spring 181 while lifting a hammer 186. At the same time, a link 183 is driven to the left, pushing test strip 127a out of slot 133 (see Figure 8). In addition, cocking actuator 140 also drives link 184 (see Figure 8) to the left, thus advancing lancet cartridge 115 into position. In this manner, actuator 140 and linkage mechanism 180 are configured to (i) cock a lancing device (*e.g.,* lift hammer 186, link 182, and spring 181) (ii) partially expose a test strip for use; and (iii) advance a lancet cartridge into position. Once actuator 140 has been cocked, button 150 can be pressed to release link 187 from under link 182, thus causing spring 181 to push link 182 and hammer 186 downward onto a lancet in cartridge 115.

One of skill in the art will appreciate that configurations of linkage mechanism 180 other that shown in the drawings can be used consistently with the inventive subject matter taught herein. In some alternative embodiments, linkage mechanism 180 is controlled and/or actuated by electrical drivers rather than pure mechanical means. For example, pulling actuator 140 could send an electrical signal to conversion electronics 137, which then operates a motor, or multiple motors, in order to perform any combination of: (i) cocking a lancing device, (ii) partially exposing a test strip for use, and (iii) advancing a lancet cartridge into position. In yet other embodiments, the lancing system is designed such that a cocking step is not required.

**Figure 10** provides a summary of the steps previously discussed in Figures 7-9. Specifically, Figure 10 shows linkage mechanism 180 coupled with lancet cartridge 115 and demonstrates four progressive stages for using linkage 180 and lancet cartridge 115 as a lancing device: initial resting position; cocking of actuator 140; cocked and ready position; and releasing and projecting a lancet. At the final stage, a finger or other body part can be placed over hole 113 in order to prick the body part and draw a blood sample.

**Figure 11** shows linkage 180 coupled with lancet cartridge 115 and test strip cartridge 125.

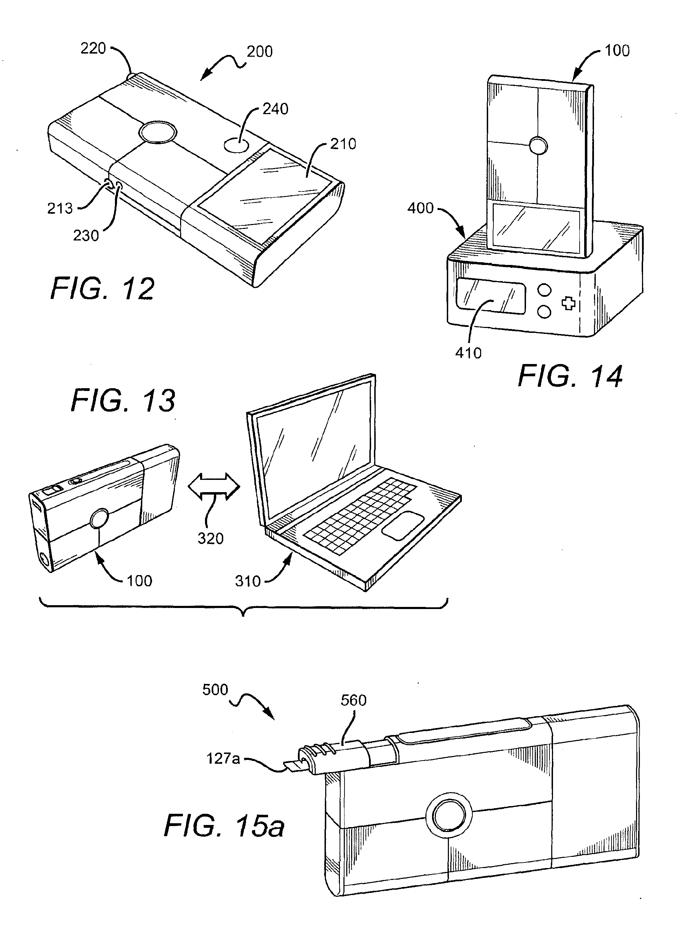

**Figure 12** shows an analyte testing device 200 having an LCD touch screen display 210. Display 210 can be used to display test results, supplies used/remaining, calories burned, time/date, history of drugs administered, journal entries, or any other number of data useful for monitoring analytes. Display 210 can also be used to type and input data into device 100.

Device 200 also has a first work light 220 positioned to illuminate a test strip that has been partially pushed out from device 200, and a second work light 230 positioned to illuminate a lancet hole 213. First and second work lights 220 and 230 are useful for using device 200 in poor lighting conditions.

Conversion electronics within device 200 additionally includes a Personal Emergency Response System (PERS), including a PERS button 240. Button 240 is configured to (i) contact a third party, (ii) identify the user of device 200, and (iii) provide a user's health data to the third-party. Examples of third parties can include spouse, relative, friend, home nurse, doctor, health care worker, ambulance operator, police operator, or any other person that can provide health care assistance. The Personal Emergency Response System is also preferably configured to automatically contact a third party as a function of the user's health data. For example, when the user is a diabetic patient, PERS can be configured to contact a third party when the user's glucose test results are below a predetermined threshold. PERS can also be configured to notify the third party of an urgency level (e.g., low, moderate, high, critical, etc), and can determine who to contact based on the urgency level.

**Figure 13** shows device 100 communicatively coupled to a laptop 310 via a wireless connection 320. Numerous wireless protocols can be used, for example Bluetooth, WiFi, 802.11, cellular, or any other protocol suitable for wireless communication. Connection 320 can be used to back up data, transmit data to a health care provider's server via the internet, reorder supplies, receive notifications from a doctor, or receive data analysis reports from an analytics software running on the laptop. It is also contemplated that connection 320 can be a wired connection. Furthermore, it is contemplated that device 100 can connect to devices other than laptop 310, for example a home computer, a smart phone, a server, or any other computing device suitable for storing, analyzing, and/or exchanging data.

**Figure 14** shows device 100 coupled with a docking station 400. Docking station 400 is configured to provide power and data connectivity to device 100. For example, station 400 can be configured to charge a re-chargeable battery within device 100. Station 400 can also be configured to back up data on device 100 and transmit data to another device, such as a home computer or a medical provider server. Docking station 400 also has a visual interface 410, through which a user can view and/or input data.

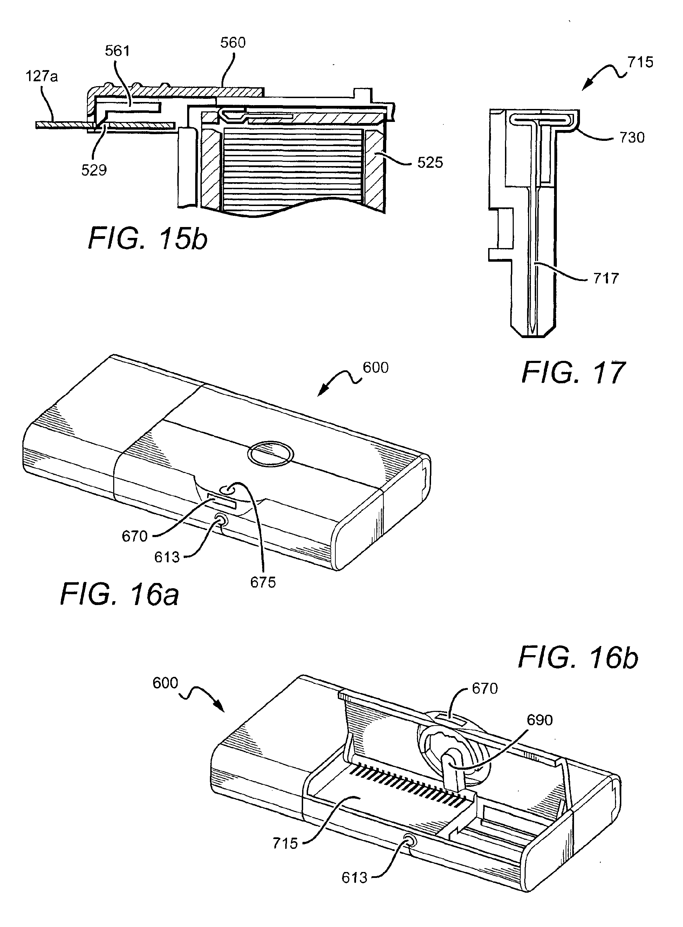

**Figure 15a** shows an analyte testing device 500, with a test strip 127a and a test strip ejector 560. **Figure 15b** is a side view cut-out of device 500, showing how ejector 560 operates to completely eject test strip 127a for disposal after usage. Ejector 560 advantageously obviates the need for direct hand contact with a used test strip. Ejector 560 has a plunger 561 configured to engage an aperture 529 of test strip 127a, thus preventing test strip 127a from being accidentally re-inserted back into cartridge 525 when a user applies finger pressure to the test strip 127 for blood application.

**Figures 16a** and **16b** show an alternate analyte testing device 600. Unlike device 100, device 600 has a wheel 670 for adjusting a lancet penetration depth. A lancet within lancet cartridge 715 exits device 600 via hole 613 according to an adjustable depth determined by the setting of wheel 670. Device 600 also has a window 675 for indicating the current lancet penetration depth setting. Spring-loaded return slider 690 is configured to retract the lancet back into cartridge 715.

**Figure 17** shows a side view of lancet cartridge 715. Unlike cartridge 115, cartridge 715 lacks a spring and cross-bar 118 for retracting lancet 717 back into cartridge 715 after lancing. Instead, lancet 717 of cartridge 715 is retracted by return slider 690 of device 600. Cartridge 715 also has a molded bump 730 that provides friction to lancet 717 and helps to maintain lancet 717 stationary when not being fired.

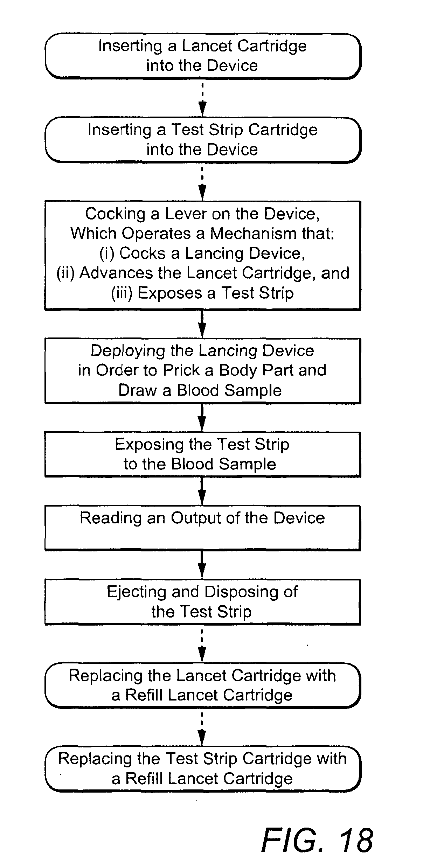

**Figure 18** shows a method of using device 100, comprising: inserting a lancet cartridge into the device; inserting a test strip cartridge into the device; cocking a lever on the device, which operates a mechanism that (i) cocks a lancing device, (ii) advances the lancet cartridge, and (iii) exposes a test strip; deploying the lancing device in order to prick a body part and draw a blood sample; and exposing the test strip to the blood sample. The first two and last two steps are displayed in round boxes and with dotted lines to indicate that these steps need not be repeated at every cycle of usage of the device. For example, in embodiments having twenty lancets and test strips per cartridge, the steps of inserting/removing cartridges need only be performed every twentieth cycle of use.

The method of Figure 18 can optionally include the steps of: reading an output of the device; ejecting and disposing of the test strip; replacing the lancet cartridge with a refill lancet cartridge; and replacing the test strip cartridge with a refill test strip cartridge.

As used herein the term "coupled to" is intended to include both direct coupling (in which two elements that are coupled to each other contact each other) and indirect coupling (in which at least one additional element is located between the two elements). Therefore, the terms "coupled to" and "coupled with" are used synonymously.

It should be apparent to those skilled in the art that many more modifications besides those already described are possible without departing from the inventive concepts herein.

## Claims

1. An analyte testing device (100), comprising:
a housing that removably holds (a) a first cartridge (115) having a plurality of lancets (117), and (b) a second cartridge (125) having a plurality of removable test strips (127);
conversion electronics (137) that receives a signal from individual ones of the plurality of test strips (127) and converts the signal into readable data; and
an actuator (140) configured to (i) cock a lancing device, (ii) expose individual ones of the plurality of test strips (127) for use; and (iii) advance the lancet cartridge (115).

2. The device of claim 1, further comprising a disengaging mechanism configured to disengage the second cartridge (125) from the actuator (140).

3. The device of claim 1, further comprising a mechanism (118,690,670) that:
retracts used lancets (117,717) into the first cartridge; or
adjusts penetration depth of the lancets (717).

4. The device of claim 1, wherein the housing has a height no more than 50 mm, a width no more than 17 mm, and a length no more than 100 mm.

5. The device of claim 1, wherein at least one of the plurality of test strips (127) is a calibration strip.

6. The device of claim 1, further comprising a first facility configured to transmit at least one of the data and information derived from the data out of the housing, using any of at least two alternative wireless protocols (320); or
a second facility configured to transmit at least one of the data and information derived from the data out of the housing over a wired path (320).

7. The device of claim 1, further comprising a processor, wherein the processor is:
programmed to correlate individual instances of at least one of the data and information derived from the data with (a) time stamps and optionally (b) user entered information selected from the group consisting of speech and text;
programmed to make an evaluation of at least one of the data and information derived from the data, and send a notification to at least one of a plurality of different recipients as a function of the evaluation;
programmed to keep track of inventory of lancets and test strips, both within and outside the device, and programmed to reorder inventory of lancets and test strips;
programmed to store voice recordings of diary information selected from the group consisting of supplies used or ordered, food eaten, exercise, medication taken, and estimated calories burned; or
programmed to prompt a user to use the device according to at least one of a selected time and a selected time interval.

8. The device of claim 1, wherein at least a first one of the plurality of test strips (127) tests for:
an analyte that is not tested for by at least a second one of the plurality of test strips;
at least two different analytes; or
glucose.

9. The device of claim 1, further comprising an LCD touch screen display (210), a first work light (230) positioned to illuminate a lancet exit hole (113,213), and a second work light (220) positioned to illuminate a test strip exit slot (133).

10. The device of claim 1, further comprising a pedometer communicatively coupled with the conversion electronics (137), and wherein the conversion electronics (137) is configured to calculate a distance traveled and an amount of calories burned associated with the distance traveled.

11. The device of claim 1, wherein the housing includes a desiccant liner configured to protect the conversion electronics (137) from external moisture.

12. The device of claim 1, wherein the conversion electronics (137) includes a personal emergency response system and a button (240) for alerting a third party of the data.

13. A method of using a blood glucose testing device, comprising:
inserting a lancet cartridge (115) into the device (100);
inserting a test strip cartridge (125) into the device (100);
cocking a lever on the device (100), which operates a mechanism that (i) cocks a lancing device, (ii) advances the lancet cartridge (115), and (iii) exposes a test strip (127);
deploying the lancing device in order to prick a body part and draw a blood sample; and
contacting the test strip (127) to the blood sample.

14. The method of claim 13, further comprising:
reading an output of the device; and
ejecting and disposing of the test strip (127).

15. The method of claim 13, further comprising:
replacing the lancet cartridge (115) with a refill lancet cartridge (115); and
replacing the test strip cartridge (125) with a refill test strip cartridge (125).

## Patentansprüche

1. Analytentestvorrichtung (100), umfassend:
eine Gehäuse, das abnehmbar (a) eine erste Kartusche (115) hält, welche mehrere Lanzetten (117) aufweist, und (b) eine zweite Kartusche (125) hält, welche mehrere herausnehmbare Teststreifen (127) aufweist;
Konversionselektronik (137), die ein Signal von einzelnen der mehreren Teststreifen (127) empfängt und das Signal in lesbare Daten umwandelt; und
einen Aktor (140), der dafür ausgelegt ist, (i) eine Stechhilfe zu spannen, (ii) einzelne der mehreren Teststreifen (127) zur Verwendung freizulegen; und (iii) die Lanzettenkartusche (115) vorzuschieben.

2. Vorrichtung nach Anspruch 1, das ferner einen Entkopplungsmechanismus umfasst, der zum Entkoppeln der zweiten Kartusche (125) vom Aktor (140) ausgelegt ist.

3. Vorrichtung nach Anspruch 1, die ferner einen Mechanismus (118, 690, 670) umfasst, der:
verwendete Lanzetten (117, 717) in die erste Kartusche zurückzieht; oder
die Eindringtiefe der Lanzetten (717) einstellt.

4. Vorrichtung nach Anspruch 1, wobei das Gehäuse eine Höhe von nicht mehr als 50 mm, eine Breite von nicht mehr als 17 mm und eine Länge von nicht mehr als 100 mm hat.

5. Vorrichtung nach Anspruch 1, wobei mindestens einer der mehreren Teststreifen (127) ein Kalibrierstreifen ist.

6. Vorrichtung nach Anspruch 1, die ferner eine erste Einrichtung umfasst, welche zum Senden von mindestens einem Element aus den Daten und Informationen ausgelegt ist, das aus den Daten aus dem Gehäuse abgeleitet ist, wobei eines von mindestens zwei alternativen drahtlosen Protokollen (320) verwendet wird; oder
eine zweite Einrichtung, die zum Senden von mindestens einem Element der Daten und Informationen ausgelegt ist, das aus den Daten aus dem Gehäuse über einen Verkabelung (320) abgeleitet wird.

7. Vorrichtung nach Anspruch 1, das ferner einen Prozessor umfasst, wobei der Prozessor folgendes ist:
programmiert zum Korrelieren einzelner Fälle von mindestens einem Element aus den Daten und Informationen, die aus den Daten mit (a) Zeitstempeln und optional (b) vom Benutzer eingegebenen Informationen abgeleitet sind, welche aus der Gruppe bestehend aus Sprache und Text ausgewählt werden;
programmiert zum Erstellen einer Bewertung von mindestens einem Element der Daten und Informationen, die aus den Daten abgeleitet wurden, und eine Meldung an mindestens einen von mehreren verschiedenen Empfängern als Funktion der Bewertung zu senden;
programmiert zum Behalten der Übersicht über den Bestand an Lanzetten und Teststreifen, beide sowohl innerhalb wie auch außerhalb der Vorrichtung, und programmiert zum Umordnen des Bestandes an Lanzetten und Teststreifen;
programmiert zum Speichern von Sprachaufzeichnungen von Tagebuchinformationen, ausgewählt aus der Gruppe bestehend aus Lieferungen, verwendet oder bestellt, Nahrung gegessen, Übungen, Medikamente genommen und geschätzte Kalorien verbrannt; oder
programmiert zum Auffordern eines Benutzers, die Vorrichtung gemäß zumindest einer gewählten Zeit und eines gewählten Zeitintervalls zu nutzen.

8. Vorrichtung nach Anspruch 1, wobei zumindest ein erster der mehreren Teststreifen (127) auf Folgendes testet:
einen Analyten, der nicht von mindestens einem zweiten der mehreren Teststreifen getestet wird; mindestens zwei unterschiedliche Analyte; oder
Glukose.

9. Vorrichtung nach Anspruch 1, die ferner ein LCD-Touchscreen-Display (210), ein erstes Arbeitslicht (230), das zum Beleuchten eines Lanzettenaustrittslochs (113, 213) positioniert ist, und ein zweites Arbeitslicht (220), das zum Beleuchten eines Teststreifenaustrittslochs (133) positioniert ist.

10. Vorrichtung nach Anspruch 1, die ferner ein Pedometer umfasst, das kommunikativ mit der Konversionselektronik (137) verbunden ist, und wobei die Konversionselektronik (137) zum Berechnen einer zurückgelegten Distanz und einer Menge an verbrannten Kalorien ausgelegt ist, welche mit der zurückgelegten Distanz verknüpft sind.

11. Vorrichtung nach Anspruch 1, wobei das Gehäuse einen Trocknungseinsatz, der zum Schützen der Konversionselektronik (137) vor äußerer Feuchtigkeit ausgelegt ist.

12. Vorrichtung nach Anspruch 1, wobei die Konversionselektronik (137) ein persönliches Notfallreaktionssystem und eine Taste (240) zum Warnen eines Dritten vor den Daten umfasst.

13. Verfahren zum Verwenden einer Blutglukosetestvorrichtung, umfassend:
Einführen einer Lanzettenkartusche (115) in die Vorrichtung (100);
Einführen einer Teststreifenkartusche (125) in die Vorrichtung (100);
Spannen eines Hebels an der Vorrichtung (100), die einen Mechanismus betätigt, welcher (i) eine Stechhilfe spannt, (ii) die Lanzettenkartusche (115) voranschiebt und (iii) einen Teststreifen (127) freilegt;
Anwenden der Stechhilfe, um einen Körperteil zu stechen und eine Blutprobe zu nehmen; und Kontaktieren des Teststreifens (127) mit der Blutprobe.

14. Verfahren nach Anspruch 13, ferner umfassend:
Lesen einer Ausgabe der Vorrichtung; und
Auswerfen und Anordnen des Teststreifens (127).

15. Verfahren nach Anspruch 13, ferner umfassend:
Ersetzen der Lanzettenkartusche (115) durch eine Nachfüll-Lanzettenkartusche (115); und Ersetzen der Teststreifenkartusche (125) durch eine Nachfüll-Teststreifenkartusche (125).

## Revendications

1. Dispositif de test d'analytes (100), comprenant :
un logement qui contient de manière amovible (a) une première cartouche (115) ayant une pluralité de lancettes (117), et (b) une seconde cartouche (125) ayant une pluralité de bandelettes de test amovibles (127) ;
une électronique de conversion (137) qui reçoit un signal de bandelettes de test individuelles de la pluralité de bandelettes de test (127) et convertit le signal en données lisibles ; et
un actionneur (140) configuré pour (i) armer un dispositif autopiqueur, (ii) exposer des bandelettes de test individuelles de la pluralité de bandelettes de test (127) pour l'utilisation ; et (iii) faire progresser la cartouche de lancettes (115).

2. Dispositif selon la revendication 1, comprenant en outre un mécanisme de libération configuré pour libérer la seconde cartouche (125) de l'actionneur (140).

3. Dispositif selon la revendication 1, comprenant en outre un mécanisme (118, 690, 670) qui :
rétracte les lancettes utilisées (117, 717) dans la première cartouche ; ou
ajuste la profondeur de pénétration des lancettes (717).

4. Dispositif selon la revendication 1, dans lequel le logement a une hauteur pas supérieure à 50 mm, une largeur pas supérieure à 17 mm, et une longueur pas supérieure à 100 mm.

5. Dispositif selon la revendication 1, dans lequel au moins une des bandelettes de test de la pluralité de bandelettes de test (127) est une bandelette d'étalonnage.

6. Dispositif selon la revendication 1, comprenant en outre un premier moyen configuré pour transmettre les données et/ou les informations dérivées des données hors du boîtier, en utilisant l'une quelconque d'au moins deux variantes de protocoles sans fil (320) ; ou
un second moyen configuré pour transmettre les données et/ou les informations dérivées des données hors du boîtier sur une trajectoire filaire (320).

7. Dispositif selon la revendication 1, comprenant en outre un processeur, dans lequel le processeur est :
programmé pour corréler des occurrences individuelles des données et/ou des informations dérivées des données à (a) des marqueurs temporels et facultativement (b) des informations entrées par l'utilisateur sélectionnées dans le groupe constitué de paroles et de texte ;
programmé pour réaliser une évaluation des données et/ou des informations dérivées des données, et envoyer une notification à au moins un destinataire d'une pluralité de destinataires différents en fonction de l'évaluation ;
programmé pour conserver un suivi de stock des lancettes et des bandelettes de test, à l'intérieur et à l'extérieur du dispositif, et programmé pour réapprovisionner le stock de lancettes et de bandelettes de test ;
programmé pour conserver des enregistrement vocaux des informations de journal sélectionnées dans le groupe constitué de fournitures utilisées ou commandées, de la nourriture mangée, des exercices, des médicaments pris, et d'une estimation des calories brûlées ; ou
programmé pour inviter un utilisateur à utiliser le dispositif en fonction d'au moins l'un d'un moment sélectionné et d'un intervalle de temps sélectionné.

8. Dispositif selon la revendication 1, dans lequel au moins une première bandelette de test d'une pluralité de bandelettes de test (127) teste :
un analyte qui n'est pas testé par au moins une seconde bandelette de test de la pluralité de bandelettes de test ;
au moins deux analytes différents ; ou
le glucose.

9. Dispositif selon la revendication 1, comprenant en outre un affichage LCD tactile (210), une première lumière de travail (230) positionnée pour éclairer un trou de sortie de lancette (113, 213), et une seconde lumière de travail (220) positionnées pour éclairer une fente de sortie de bandelettes de test (133).

10. Dispositif selon la revendication 1, comprenant en outre un pédomètre couplé de façon à pouvoir communiquer avec l'électronique de conversion (137), et dans lequel l'électronique de conversion (137) est configurée pour calculer une distance parcourue et une quantité de calories brûlées associée à la distance parcourue.

11. Dispositif selon la revendication 1, dans lequel le logement comprend un revêtement dessicatif configuré pour protéger l'électronique de conversion (137) de l'humidité externe.

12. Dispositif selon la revendication 1, dans lequel l'électronique de conversion (137) comprend un système de réponse d'urgence personnel et un bouton (240) pour alerter une tierce partie des données.

13. Procédé d'utilisation d'un dispositif de test de glycémie, comprenant :
l'insertion d'une cartouche de lancettes (115) dans le dispositif (100) ;
l'insertion d'une cartouche de bandelettes de test (125) dans le dispositif (100) ;
l'armement d'un levier sur le dispositif (100), qui actionne un mécanisme qui (i) arme un dispositif autopiqueur, (ii) fait progresser la cartouche de lancettes (115), et (iii) expose une bandelette de test (127) ;
le déploiement du dispositif autopiqueur en vue de piquer une partie du corps et de prélever un échantillon de sang ; et
la mise en contact de la bandelette de test (127) avec l'échantillon de sang.

14. Procédé selon la revendication 13, comprenant en outre :
la lecture d'un résultat du dispositif ; et
l'éjection et l'élimination de la bandelette de test (127).

15. Procédé selon la revendication 13, comprenant en outre :
le remplacement de la cartouche de lancettes (115) avec une recharge de cartouche de lancettes (115) ; et
le remplacement de la cartouche de bandelettes de test (125) avec une recharge de cartouche de bandelettes de test (125).
